# EUROPEAN PATENT APPLICATION

(11) **EP 4 417 693 A1**
(43) Date of publication of application: **21.08.2024**
(21) Application number: 22880979.4
(22) Date of filing: 11.10.2022
(51) Int. Cl.: C12N 15/113, A61K 31/7052, A61P 43/00, C12N 5/0775, C12N 5/10

(54) **EXTRACELLULAR VESICLE SECRETION BOOSTER FOR BOOSTING EXTRACELLULAR VESICLE SECRETION, AND USE THEREOF**

(30) Priority: 11.10.2021 JP 2021166634
(71) Applicant: THEORIA Science Inc., Tokyo 101-0062 (JP)
(72) Inventor: OCHIYA, Takahiro, Tokyo 101-0062 (JP); YAMAMOTO, Tomofumi, Tokyo 101-0062 (JP)
(74) Representative: J A Kemp LLP
(86) International application number: PCT/JP2022/037768
(87) International publication number: WO 2023/063284

(57) **Abstract**

Provided are a novel secretion booster and secretion boosting method with respect to extracellular vesicle secretion from cells. The extracellular vesicle secretion booster of the present invention contains an ABCA1 reducing agent. The extracellular vesicle secretion boosting method of the present invention includes boosting extracellular vesicle secretion by causing a target having a cell to coexist with the extracellular vesicle secretion booster of the present invention.

## Description

### Technical Field

The present invention relates to an extracellular vesicle secretion booster for boosting extracellular vesicle secretion from cells and the use thereof.

### Background Art

Various functions of extracellular vesicles such as exosomes secreted from cells have been reported, and the use thereof is attracting attention. Such extracellular vesicles contain, for example, nucleic acids, such as microRNA (miRNA) and proteins. The extracellular vesicles mediate the transmission of their inclusions from a cell that has secreted these extracellular vesicles to a recipient cell, and thus are considered to function as a cell-to-cell communication tool. The same function has also attracted attention to the use thereof as carriers in drug delivery systems (DDSs).

### Summary of the Invention

### Technical Problem

However, the mechanism of their secretion, namely, how the extracellular vesicle secretion from cells is regulated, has not yet been clarified. Therefore, for example, even if an attempt is made to produce the extracellular vesicles as a material that can be used as drugs for diseases or carriers in DDSs, their efficient production is difficult. Furthermore, even if an attempt is made to obtain a therapeutic effect by causing endogenous EV secretion in a living organism, a method for effectively boosting their secretion has not been established.

In light of the foregoing, it is an object of the present invention to provide a novel secretion booster and secretion boosting method with respect to extracellular vesicle secretion from cells.

### Solution to the Problem

In order to achieve the above-described object, the present invention provides an extracellular vesicle secretion booster containing an ABCA1 reducing agent.

The present invention also provides a secretion boosting method including boosting extracellular vesicle secretion by causing a target having a cell to coexist with the extracellular vesicle secretion booster of the present invention.

The present invention also provides a method for producing an extracellular vesicle, including: boosting extracellular vesicle secretion by causing a cell to coexist with the extracellular vesicle secretion booster of the present invention; and collecting an extracellular vesicle secreted from the cell.

### Advantageous Effects of Invention

According to the extracellular vesicle secretion booster of the present invention, extracellular vesicle secretion from cells can be boosted. Thus, for example, the preparation of extracellular vesicles as a material and the secretion of endogenous extracellular vesicles in a living organism by causing extracellular vesicle secretion from cells can be efficiently performed. Therefore, the present invention can be said to be a very useful technology, for example, in medical and other various fields.

### Brief Description of Drawings

[FIG. 1] FIG. 1 shows graphs of the amount of EVs secreted from HCT116 cells transfected with miR-3202.
[FIG. 2] FIG. 2 shows graphs of the amount of EVs secreted from A549 cells transfected with miR-3202.
[FIG. 3] FIG. 3 shows graphs of the amount of EVs secreted from a plurality of types of cells transfected with miR-3202.
[FIG. 4] FIG. 4 shows graphs of the particle size distribution of EVs secreted from HCT116 cells transfected with miR-3202.
[FIG. 5] FIG. 5 shows graphs of the particle size distribution of EVs secreted from A549 cells transfected with miR-3202.
[FIG. 6] FIG. 6 shows a graph of the caspase activity of a plurality of types of cells transfected with miR-3202.
[FIG. 7] FIG. 7 shows graphs of the amount of EVs secreted from MSC cells transfected with miR-3202.
[FIG. 8] FIG. 8 shows graphs of the particle size distribution of EVs secreted from MSC cells transfected with miR-3202.
[FIG. 9] FIG. 9 shows graphs of the relative values of the expression level of ABCA1 mRNAin cells transfected with siRNA against the ABCA1 gene.
[FIG. 10] FIG. 10 shows results of cells transfected with siRNA against the ABCA1 gene, where the upper graphs are graphs of the cell viability, and the lower graphs are graphs of the relative values of the amount of secreted EVs.
[FIG. 11] FIG. 11 shows results of A549 cells to which ABCA1 inhibitors were added, where the upper graph is a graph of the cell viability, and the lower graph is a graph of the relative values of the amount of secreted EVs.
[FIG. 12] FIG. 12 shows results of HCT116 cells to which ABCA1 inhibitors were added, where the upper graph is a graph of the cell viability, and the lower graph is a graph of the relative values of the amount of secreted EVs.

### Description of Embodiments

In the present invention, extracellular vesicles are hereinafter also referred to as EVs. An extracellular vesicle secretion booster of the present invention is also hereinafter referred to as an EV secretion booster.

The extracellular vesicle secretion booster of the present invention is characterized by, as described above, containing an ABCA1 reducing agent.

In the EV secretion booster of the present invention, for example, the ABCA1 reducing agent is an expression reducing substance or a function reducing substance for(against) an ABCA1 protein.

In the EV secretion booster of the present invention, for example, the expression reducing substance is at least one selected from the group consisting of substances that reduce transcription from a gene encoding an ABCA1 protein, substances that degrade a transcription product resulting from transcription, and substances that reduce translation of the transcription product into a protein.

In the EV secretion booster of the present invention, for example, the expression reducing substance is at least one nucleic acid substance selected from the group consisting of miRNAs, siRNAs, antisenses, and ribozymes, and precursors thereof.

In the EV secretion booster of the present invention, for example, the expression reducing substance is an expression vector for expression of the nucleic acid substance.

In the EV secretion booster of the present invention, for example, the expression reducing substance is miR-3202 or a precursor thereof.

In the EV secretion booster of the present invention, for example, the precursor is at least one selected from the group consisting of a nucleic acid encoding the miRNAs, a primary miRNA transcription product, a precursor miRNA, and an expression vector of the miRNAs.

In the EV secretion booster of the present invention, for example, the function reducing substance is a function inhibitory substance or a function neutralizing substance for(against) an ABCA1 protein.

In the EV secretion booster of the present invention, for example, the function neutralizing substance is an antibody or antigen-binding fragment against an ABCA1 protein.

In the EV secretion booster of the present invention, for example, the function reducing substance is an expression vector for expression of the function neutralizing substance.

The EV secretion booster of the present invention is, for example, for boosting extracellular vesicle secretion from a cell, and the cell is a mesenchymal stem cell.

The secretion boosting method of the present invention is characterized by, as described above, including: boosting extracellular vesicle secretion by causing a target having a cell to coexist with the extracellular vesicle secretion booster of the present invention.

In the EV secretion boosting method of the present invention, for example, in the boosting, the extracellular vesicle secretion booster is added to the target in vivo, ex vivo, or in vitro.

In the EV secretion boosting method of the present invention, for example, in the boosting, the cell is transfected with the extracellular vesicle secretion booster.

In the EV secretion boosting method of the present invention, for example, the cell is a mesenchymal stem cell.

In the EV secretion boosting method of the present invention, for example, the cell is a mesenchymal stem cell derived from bone marrow or adipose tissue.

In the EV secretion boosting method of the present invention, for example, the cell is a cell collected from a living organism or a cell of a cell line.

In the EV secretion boosting method of the present invention, for example, in the boosting, the cell is caused to coexist with the extracellular vesicle secretion booster in a medium.

In the EV secretion boosting method of the present invention, for example, the target is tissue, an organ, or a living organism.

The method for producing an extracellular vesicle of the present invention is characterized by, as described above, including: boosting extracellular vesicle secretion by causing a cell to coexist with the extracellular vesicle secretion booster of the present invention; and collecting an extracellular vesicle secreted from the cell.

In the EV production method of the present invention, for example, the cell is transfected with the extracellular vesicle secretion booster.

In the EV production method of the present invention, for example, the cell is a mesenchymal stem cell.

In the EV production method of the present invention, for example, the cell is a mesenchymal stem cell derived from bone marrow or adipose tissue.

In the EV production method of the present invention, for example, the cell is a cell collected from a living organism or a cell of a cell line.

In the EV production method of the present invention, for example, in the boosting, the cell is caused to coexist with the extracellular vesicle secretion booster in a medium.

The EV production method of the present invention is, for example, for producing the extracellular vesicle as a carrier in a drug delivery system.

The EV production method of the present invention is, for example, for producing the extracellular vesicle as a therapeutic agent.

### <EV Secretion Booster>

The EV secretion booster (promotor) of the present invention is characterized by, as described above, containing an ABCA1 reducing agent. The EV secretion booster of the present invention is characterized by containing the ABCA1 reducing agent, and other configurations and conditions are not particularly limited. As to the EV secretion booster of the present invention, reference can be made to the following descriptions regarding the EV secretion boosting (promoting) method and the like of the present invention.

The present invention is characterized by being based on the finding that the ABCA1 is involved in EV secretion and that the EV secretion from cells can be reduced by reducing the ABCA1. Thus, the type of substance used for reducing the ABCA1 and the method for reducing the same are not limited by any means.

ABCA1 stands for ATP-binding cassette transporter 1 (member 1 of human transporter sub-family ABCA). ABCA1 protein and ABCA1 gene encoding the same are registered under Gene ID: 19 in the Genetic Testing Registry (GTR) database.

In the present invention, the "ABCA1 reducing agent" may be any agent that can reduce the ABCA1, and it may reduce, for example, the expression of an ABCA1 protein or the function of the ABCA1 protein. In the former case, the ABCA1 reducing agent is, for example, an expression reducing substance for an ABCA1 protein, and, in the latter case, the ABCA1 reducing agent is, for example, a function reducing substance for an ABCA1 protein. In the present invention, "reduce" may mean "inhibit".

The EV secretion booster of the present invention may contain, as the active ingredient, for example, only the above-described ABCA1 reducing agent or may further contain other ingredients for boosting the EV secretion in addition to the ABCA1 reducing agent. The EV secretion booster of the present invention may contain, for example, any one of or both the expression reducing substance and the function reducing substance, as the ABCA1 reducing agent.

The type of ABCA1 reducing agent is not particularly limited, and it may be, for example, a low molecular weight compound such as a nucleic acid substance or an organic compound, a protein such as an antibody, a peptide such as an antigen-binding fragment, or the like.

The expression reducing substance is not particularly limited, and may be, for example, a substance that reduces either transcription or translation during expression of an ABCA1 protein (hereinafter also referred to as a "target protein") from the gene (hereinafter also referred to as an "ABCA1 gene" or a "target gene") encoding the ABCA1 protein. Examples of the reduction of transcription include inhibition of transcription from DNA to an mRNA precursor, inhibition of RNA processing to form a mature mRNA from an mRNA precursor, and degradation of an mRNA precursor or a mature mRNA. Examples of the reduction of translation include inhibition of translation from a mature mRNA and inhibition of modification of a translation product.

The expression reducing substance is, for example, a nucleic acid substance (hereinafter also referred to as a "nucleic acid-type reducing substance"), and may be embodied as a nucleic acid substance that reduces the expression as it is (first embodiment) or may be embodied in the form of a precursor that converts into a state capable of reducing the expression when it is in vivo, in vitro, or ex vivo (second embodiment).

The expression reducing substance of the first embodiment is, for example, an antigene substance, an antisense (antisense oligonucleotide), an RNA interference (RNAi) substance, or a ribozyme. The RNAi substance is, for example, siRNA or miRNA. The antigene substance inhibits, for example, mRNA transcription. The antisense and miRNA inhibit, for example, translation from mRNA. The siRNA and ribozyme, for example, degrade mRNA. These expression reducing substances may target, for example, either the entire region or a partial region of the target gene. As specific examples, the antisense and miRNA can be designed, for example, so as to bind to the 3' UTR region of mRNA transcribed from the target gene, and the siRNA and ribozyme can be designed, for example, so as to fully complementarily bind to a partial region of mRNA transcribed from the target gene.

Specific examples of the miRNA include miR-3202. The miRNA is, for example, registered under the following accession number in the NCBI DB (https://www.ncbi.nlm.nih.gov/nuccore).
miR-3202: No. NR_036175

The miRNAs in the active ingredient may be used, for example, alone or in a combination of two or more. The miRNAs (microRNAs) are, for example, single-stranded RNA molecules.

The expression reducing substance of the first embodiment can be, for example, obtained by a screening method to be described below or can be designed from the sequence of the target gene.

The expression reducing substance of the first embodiment may be, for example, either a single strand or a double strand. The structural units of the expression reducing substance are not particularly limited. Examples of the structural units include a deoxyribonucleotide backbone and a ribonucleotide backbone, each including a sugar, a base such as purine or pyrimidine, and phosphoric acid. Other examples of the structural units include non-nucleotide backbones including a base such as pyrrolidine or piperidine. These backbones may be either modified or unmodified. The structural units may be, for example, natural structural units or unnatural, i.e., artificial structural units. The expression reducing substance may be, for example, composed of the same structural units or two or more types of structural units.

The expression reducing substance of the second embodiment is, as described above, the precursor, and specific examples thereof include precursors that express an expression reducing substance of the first embodiment. By administering the precursor to a target, the expression reducing substance of the first embodiment can be expressed and allowed to function in, for example, an in vivo, in vitro, or ex vivo environment.

The precursor may be, for example, in a form that includes the expression reducing substance of the first embodiment and a linker. As a specific example, the precursor may be, for example, in a form in which both strands of siRNA are linked together via the linker. The precursor in such a form can generate (express) a double-stranded siRNA when, for example, the linker is removed from the precursor upon cleavage of the precursor in an in vivo, in vitro, or ex vivo environment. A specific example of the precursor is shRNA that generates siRNAs when, for example, it is cleaved.

As a specific example, the type of miRNA precursor is not particularly limited, and it may be, for example, any substance that generates the miRNAs in cells. Examples of the precursor include a nucleic acid encoding the miRNAs, a primary miRNA transcription product (pri-miRNA), and a precursor miRNA (pre-miRNA).

Alternatively, the precursor may be, for example, an expression vector with the coding sequence of the expression reducing substance of the first embodiment inserted therein. For example, the coding sequence of the precursor (e.g., the above-mentioned shRNA, etc.) may be inserted in the expression vector. Such an expression vector can cause the expression of the expression reducing substance of the first embodiment in, for example, an in vivo, in vitro, or ex vivo environment. The type of expression vector is not particularly limited, and it may be, for example, a plasmid vector or a viral vector. Examples of the viral vector include adenovirus vectors and Sendai virus vectors.

Specific examples of the expression vector include an expression vector of the miRNAs. The coding sequence in the expression vector may be, for example, a gene that generates pri-miRNA through transcription. The expression vector may be, for example, an expression vector with the coding sequence of miRNA inserted in the cloning site of miRNA in the vector. The coding sequence of the miRNA is, for example, a coding sequence of a single-stranded pri-miRNA or pre-miRNA having a stem-loop structure.

The function reducing substance may be, for example, a function inhibitory substance that inhibits the function of the ABCA1 protein (also referred to as an "activity inhibitory substance") or a function neutralizing substance that neutralizes the function of the protein (also referred to as an "activity neutralizing substance").

The function inhibitory substance is not particularly limited, and may be a low molecular weight compound or the like. Specific examples thereof include cyclosporin, probucol, valspodar, and their analogs.

The function neutralizing substance may be, for example, an antibody or antigen-binding fragment (antigen-binding peptide) against the ABCA1 protein (such an antibody and antigen-binding fragment are also collectively referred to as "antibody-type reducing substances" hereinafter). Since the antibody-type reducing substance can reduce the function of the ABCA1 protein by, for example, binding to the ABCA1 protein, it can also be said to be a neutralizing antibody or a neutralizing antigen-binding fragment. The antibody-type reducing substance can also be obtained by, for example, a screening method to be described below.

The antibody may be, for example, either a monoclonal antibody or a polyclonal antibody. The isotype thereof is not particularly limited, and may be, for example, IgG, IgM, or IgA. In the case where the antibody is administered to a human, it preferably is, for example, a fully human antibody, a humanized antibody, or a chimeric antibody.

The antigen-binding fragment need only be capable of, for example, recognizing a target site of the target protein and binding thereto, and examples of the antigen-binding fragment include fragments that include a complementarity-determining region (CDR) of the antibody. Specific examples of the antigen-binding fragment include fragments such as Fab, Fab', and F(ab').

The function reducing substance may be, for example, of the first embodiment in which the function reducing substance reduces the function of the ABCA1 protein as is, or may be of the second embodiment in which the function reducing substance is a precursor that converts into a state capable of reducing the function of the ABCA1 protein in an in vivo, in vitro, or ex vivo environment. The function reducing substance of the first embodiment is, for example, the above-described antibody-type reducing substance. The precursor of the second embodiment may be, for example, an expression vector with the coding sequence of a protein (e.g., an antibody) or a peptide (e.g., an antigen-binding fragment) that reduces the function of the ABCA1 protein inserted therein. The type of expression vector is not particularly limited, and it may be, for example, a plasmid vector or a viral vector, as with the expression vector described above.

The function reducing substance may be, for example, a substance that allows the ABCA1 protein to have its function and to maintain the function but reduces the conditions under which the ABCA1 protein can exhibit the function. As a specific example, the function reducing substance may be a reducing substance that reduces a substance (also referred to as a "substrate") that the ABCA1 protein needs in order to exhibit its function or a reducing substance that alters the substrate. The reduction of the substrate may be achieved by, for example, reducing the generation of the substrate or degrading the substrate.

The EV secretion booster of the present invention may contain, for example, only the above-described active ingredient or may further contain other additive ingredients. The EV secretion booster of the present invention may contain, as the active ingredient, for example, only the above-described ABCA1 reducing agent or may further contain other active ingredients involved in the boosting of EV secretion in addition to the ABCA1 reducing agent. The additive ingredients are not particularly limited, and examples thereof include ingredients to be described below, which are preferably pharmacologically acceptable. The additive ingredients can be set as appropriate according to, for example, the method for administering the EV secretion booster, the administration target, and the dosage form.

One example of the additive ingredients is a vehicle. Examples of the vehicle include liquid media such as aqueous solvents, alcohol solvents, polyalcohol solvents, lipid solvents, and mixed solvents thereof (e.g., emulsifying solvents), lactose, and starch. Examples of the aqueous solvents include water, physiological saline, and isotonic solutions containing sodium chloride and the like. Examples of the lipid solvents include soybean oil. Other examples of the additive ingredients include: binding agents such as starch glue; disintegrants such as starch and carbonate; and lubricants such as talc and wax. The additive ingredients may also include, for example, a DDS agent for delivering the active ingredient to a target site.

The additive ingredients may be, for example, a transfection agent for transfecting cells with the ABCA1 reducing agent in the present invention. The additive ingredients may be, for example, a DDS agent for delivering the ABCA1 reducing agent in the present invention to target cells of a target.

The additive ingredients are, for example, preferably pharmacologically acceptable ingredients. The additive ingredients can be set as appropriate according to, for example, the method for supplying the EV secretion booster of the present invention to a target, the type of the target, and the dosage form of the EV secretion booster.

According to the EV secretion booster of the present invention, EV secretion from cells can be boosted. In the present invention, cells to be subjected to boosting of EV secretion are not particularly limited, and may be various cells derived from a human or a non-human animal. The cells may be, for example, normal cells or cells that are abnormal for an item of interest.

In the present invention, extracellular vesicles (EVs) are, for example, exosomes, microvesicles (submicroscopic vesicles), and apoptotic bodies secreted through endocytosis pathways. In particular, the extracellular vesicles in the present invention, for example, are exosomes. In general, exosomes can be detected using a marker molecule such as Alix, Tsg101, CD81, CD63, CD9, or flotillin.

It is known that the EVs contain an information cargo such as a protein or an RNA, and, when secreted from a cell, they have the function of mediating cell-to-cell information transmission through translocation to another cell. Based on such a function, it has been reported that EVs secreted from cells show therapeutic effects on diseases at the cellular level. In addition, the use of secreted EVs for drug delivery, for example, as carriers in drug delivery systems, has also attracted attention. Thus, with the EV secretion booster of the present invention, for example, EV secretion from cells can be boosted by supplying the booster to target cells in vivo, ex vivo, or in vitro, depending on the purpose. The use of the EV secretion booster of the present invention will be described in more detail, for example, in the EV secretion boosting method of the present invention and the EV production method of the present invention below, and reference can be made thereto.

### <EV Secretion Boosting Method>

The EV secretion boosting method of the present invention is characterized by, as described above, including: boosting extracellular vesicle secretion by causing a target having cells to coexist with the EV secretion booster of the present invention. The EV secretion boosting method of the present invention can boost the extracellular vesicle secretion from cells of the target, and thus it can also be said to be an "EV production method".

The target is not particularly limited, and, in the EV secretion boosting method of the present invention, for example, the EV secretion booster of the present invention may be supplied to a target having cells to be subjected to boosting of EV secretion. The type of cells is not particularly limited, and they may be, as a specific example, mesenchymal stem cells or epithelial cells. The source of the mesenchymal stem cells is not particularly limited, and it may be, for example, bone marrow, umbilical cord, dental pulp, or adipose tissue. The cells may be, for example, normal cells or abnormal cells.

The method for supplying the EV secretion booster of the present invention is not particularly limited, and the EV secretion booster may be, for example, supplied in vitro, ex vivo, or in vivo.

The target to which the EV secretion booster of the present invention is to be supplied in vitro is, for example, cells, and the cells may be, for example, cells collected (isolated) from a living organism or cells of cell lines, or a culture thereof. The target to which the EV secretion booster of the present invention is to be supplied ex vivo is, for example, tissue composed of the cells as structural units, an organ composed of the tissue as structural units, or the like, and the tissue or the organ is isolated, for example, from a living organism. The target to which the EV secretion booster of the present invention is to be supplied in vivo is, for example, a living organism. The source and type of cells, tissue, or organ are not particularly limited, and they may be, for example, fat, large intestine, lung, skin, breast, breast duct, mammary gland, pancreas, or bone marrow.

The cells, the tissue, and the organ may be, for example, derived from a human or a non-human animal. The living organism may be, for example, a human or a non-human animal. Examples of the non-human animal include mammals such as mice, rats, rabbits, horses, sheep, cows, and camels. When the target is derived from a non-human animal or is a non-human animal, a preferable ABCA1 reducing agent is, for example, a reducing substance that relatively specifically acts on an ABCA1 protein or ABCA1 gene derived from this particular non-human animal. When the target to which the ABCA1 reducing agent is to be added is derived from a human or is a human, a preferable ABCA1 reducing agent is, for example, a reducing substance that relatively specifically acts on an ABCA1 protein or ABCA1 gene derived from a human.

The type of the cells, the tissue, and the organ is not particularly limited, and any target cells to be subjected to boosting of EV secretion can be selected. The cells, the tissue, and the organ may be, for example, those mentioned as examples above.

The EV secretion boosting method of the present invention may, for example, further include collecting EVs secreted from the cells. The collecting of EVs can also be said to be, for example, isolating of EVs secreted from the cells.

Specific examples of the EV secretion boosting method of the present invention are given below, but the invention is not limited in any way to these examples.

As mentioned above, EVs have recently been reported for their potential use in the treatment of various diseases. For example, in the case of EVs from mesenchymal stem cells, their therapeutic effects on immunological diseases, ischemic diseases, inflammatory diseases, neurological diseases, diabetes mellitus, and the like have been reported. In this manner, since the EVs derived from mesenchymal stem cells can be an active ingredient of therapeutic agents for these diseases, it is possible to obtain efficiently EVs that can be used as an active ingredient by boosting the EV secretion from mesenchymal stem cells using the EV secretion booster of the present invention. The source of the mesenchymal stem cells is not particularly limited, and it may be, for example, those mentioned as examples above. When using the EVs as an active ingredient in the treatment of the diseases, preferable mesenchymal stem cells are, for example, normal cells.

Other possible sources of EVs that can be an active ingredient of therapeutic agents may be, for example, normal epithelial cells (Kadota et al., JEV, 2021) or the like as a specific example, but the present invention is not limited thereto.

Furthermore, the EVs are, for example, known to contain a substance as described above and to mediate cell-to-cell information transmission. Therefore, the EVs can be used, for example, as a carrier in a drug delivery system. Thus, for example, the EVs that can be used as the carrier can be efficiently prepared by boosting the EV secretion from target cells using the EV secretion booster of the present invention. The target cells to be subjected to boosting of secretion of the EVs that can be used as the carrier are not particularly limited, and may be, for example, mesenchymal stem cells or the like, and preferable cells as such cells are, for example, normal cells.

Furthermore, also in the case of the abnormal cells involved in various diseases, , for example, by causing the cells to coexist with the EV secretion booster of the present invention, the EV secretion can be boosted, and the EVs can be collected. In studying the diseases and the abnormal cells, EVs derived from the abnormal cells can be a subject for an analysis, and the present invention can be used to efficiently obtain samples for the analysis.

In the method for boosting the EV secretion from cells in a target using the EV secretion booster of the present invention, it is sufficient that the target and the EV secretion booster are co-present. Specific examples are given below, but the invention is not limited to these examples. As to each of the following embodiments, reference can be made to the descriptions regarding the other embodiments, unless otherwise stated.

### (Embodiment 1)

When supplying the EV secretion booster of the present invention in vitro, for example, it is preferable to cause the cells to coexist with the EV secretion booster in a medium, and to incubate the cells in the medium in the presence of the EV secretion booster. This allows, for example, EVs to be secreted from the cells into the medium, and thus the secreted EVs can be easily collected from the medium. The incubation of the cells in the medium may be, for example, a two-dimensional culture of the cells or a three-dimensional culture of the cells using a scaffold or the like.

The cells to be caused to coexist with the EV secretion booster of the present invention may be, for example, uncultured cells or cultured cells, preferably the latter. Specifically, for example, it is preferable to pre-culture the cells and then perform the main culture of the cells in the presence of the EV secretion booster in the medium to boost secretion of EVs from the cells.

Although the EV secretion booster of the present invention may, for example, be simply caused to coexist with the cells, the cells are preferably transfected with the ABCA1 reducing agent in the present invention. In this case, the EV secretion booster of the present invention is used, for example, further in the presence of a transfection agent (also referred to as a "gene transfer agent"). The type of the transfection agent is not particularly limited, and, for example, commercially available reagents such as LIPOFECTAMINE^{®} can be used. The conditions for the addition of the transfection agent are not particularly limited, and they can be set as appropriate according to, for example, the type of cells, the number of cells, the type of medium, the amount of medium, and the like.

The incubation conditions (e.g., medium, temperature, time, humidity, etc.) for the cells are not particularly limited, and they can be set as appropriate according to, for example, the type of cells, the type of medium, and the like. As a specific example, if the cells are mesenchymal stem cells, the incubation conditions are set to, for example, MesenPRO RS medium, culture time of 24 to 96 hours, and culture temperature at 36 to 37°C. Similar conditions can be employed, for example, for other cells as well.

The amount of the EV secretion booster of the present invention added to the cells is not particularly limited. As a specific example, if the EV secretion booster is the expression reducing substance (e.g., RNAi substances such as miRNA, siRNA, etc.), it is added to the medium, for example, to a final concentration of 25 to 100 nmol/L.

As a specific example, if the EV secretion booster is the function reducing substance (e.g., a function inhibitory substance such as the above-mentioned low molecular weight compound, etc.), it is added to the medium, for example, to a final concentration of 10 to 100 nmol/L.

The EV secretion boosting method of this embodiment may further include, for example, collecting EVs secreted from the cells. The collecting of EVs can also be said to be, for example, isolating of EVs secreted from the cells.

The method for collecting EVs is not particularly limited. For example, after the cells are incubated in the medium in the presence of the EV secretion booster, the cells may be removed from the medium and a liquid fraction such as supernatant may be collected, and then EVs may be collected from the liquid fraction. For example, filtration such as ultrafiltration, centrifugation such as ultracentrifugation, and the like can be used to collect EVs from the supernatant.

### (Embodiment 2)

As to the EV secretion booster of the present invention, reference can be made to the above descriptions regarding the supply to the cells in vitro, even in the case in which the supply is performed ex vivo and the target is the tissue or the organ. The incubation conditions are not particularly limited either, and they can be set as appropriate according to, for example, the type of tissue or organ, the size thereof, and the like. As to the amount of the EV secretion booster of the present invention that is added, for example, reference can be made to the above-mentioned example regarding the amount of booster added to the cells.

The EV secretion boosting method of this embodiment may further include, for example, collecting EVs secreted from cells of the tissue or the organ. The collecting of EVs can also be said to be, for example, isolating of EVs secreted from the cells.

The method for collecting EVs is not particularly limited. For example, after the tissue or the organ is incubated in the medium in the presence of the EV secretion booster, the tissue or the organ may be removed from the medium and a liquid fraction such as supernatant may be collected, and then EVs may be collected from the liquid fraction. For example, filtration such as ultrafiltration, centrifugation such as ultracentrifugation, and the like can be used to collect EVs from the supernatant.

### (Embodiment 3)

If the EV secretion booster of the present invention is supplied in vivo, the target may be, for example, a living organism having the cells. The living organism may be, for example, a human or a non-human animal. Examples of the non-human animal include mammals such as mice, rats, rabbits, horses, sheep, cows, and camels.

As mentioned above, EVs have been reported to have therapeutic effects on various diseases. Thus, for example, therapeutic effects can be obtained by administering the EV secretion booster of the present invention to a living organism to boost EV secretion from cells.

The method for administering the EV secretion booster of the present invention is not particularly limited, and it may be parenteral administration, oral administration, intravenous administration, or the like. The administration conditions are not particularly limited, and they can be determined as appropriate according to, for example, the type of living organism and the type of cells, tissue, or organ as the administration target, and the like. As to the amount of the EV secretion booster of the present invention added, for example, reference can be made to the above-mentioned example regarding the amount of booster added to the cells.

When the administration method is parenteral administration, an administration site may be, for example, target tissue or a target organ, i.e., tissue or an organ that includes cells to be subjected to boosting of EV secretion (such tissue or an organ is also referred to as a "target site"), or a site from which the ABCA1 reducing agent in the present invention as the active ingredient of the EV secretion booster can be delivered to the target site. As a specific example, when the target cells are, for example, large intestinal cells, the administration site may be the large intestine as the target site or a site from which the ABCA1 reducing agent in the present invention can be delivered to the large intestine. When the target cells are, for example, lung cells, the administration site may be the lung as the target site, or a site from which the ABCA1 reducing agent in the present invention can be delivered to the lung. The same applies to other organs. Examples of the parenteral administration method include injection to an affected area, intravenous injection, subcutaneous injection, intradermal injection, intravenous infusion, and transdermal administration. The form of the EV secretion booster of the present invention is not particularly limited, and it can be set as appropriate according to the administration method and the like, as described above. As to the form of the EV secretion booster, reference can be made to the above descriptions therein.

In the case of parenteral administration, the dosage form of the EV secretion booster of the present invention is not particularly limited, and it can be determined as appropriate according to the administration method. For example, the EV secretion booster may be in the form of liquid, cream, or gel, and can be prepared by mixing the ABCA1 reducing agent in the present invention with a medium. Of the above-described examples of the medium, the aqueous solvent is, for example, physiological saline or an isotonic solution, the lipid solvent is, for example, soybean oil, and the emulsifying solvent is, for example, a mixture thereof. Such a parenteral administration agent may further contain, for example, alcohol, polyalcohol, and/or a surfactant. Also, in the case of parenteral administration, the EV secretion booster of the present invention may contain, for example, a DDS agent for effectively delivering the ABCA1 reducing agent in the present invention from a site other than a target site, to the target site. In particular, in order to effectively deliver the ABCA1 reducing agent in the present invention to, for example, particular cells (e.g., cancer cells) in tissue as the target site, the EV secretion booster of the present invention may contain, for example, a DDS agent that specifically recognizes the particular cells.

In the case of oral administration, the dosage form of the EV secretion booster of the present invention is not particularly limited, and examples thereof include tablets, pills, granules, powder medicines, capsules, and syrups. The EV secretion booster of the present invention may contain, for example, a diluent, a vehicle, and/or a carrier. The EV secretion booster of the present invention may also contain, for example, a DDS agent for effectively delivering the ABCA1 reducing agent in the present invention to the target site. In particular, in order to effectively deliver the ABCA1 reducing agent in the present invention to, for example, particular cells (e.g., cancer cells) in tissue as the target site, the EV secretion booster of the present invention may contain, for example, a DDS agent that specifically recognizes the particular cells.

As to administration to a living organism, the administration conditions of the EV secretion booster of the present invention can be determined as appropriate according to, for example, the age, the body weight, the type of tissue or organ as the administration target, and the sex.

As described above, EVs are known to have therapeutic effects on various diseases. Thus, secretion of EVs having such effects can be boosted by administering the EV secretion booster of the present invention to a living organism. Thus, the EV secretion booster of the present invention itself can also be said to be, for example, a pharmaceutical composition that indirectly treats the above-mentioned diseases.

Furthermore, as described above, the EVs are known to contain, for example, an information cargo in a cell, and to mediate cell-to-cell information transmission through translocation. Thus, the EV secretion booster of the present invention can also be said to be, for example, a cell-to-cell information transmission booster, and the EV secretion boosting method of the present invention can also be said to be, for example, a cell-to-cell information transmission boosting method.

### <EV Production Method>

The EV production method of the present invention is characterized by, as described above, including: boosting extracellular vesicle secretion by causing cells to coexist with the EV secretion booster of the present invention; and collecting an extracellular vesicle secreted from the cells. As to the EV secretion booster and the boosting of the present invention for use in the EV production method of the present invention, reference can be made to the above descriptions regarding the EV secretion booster of the present invention and the EV secretion boosting method of the present invention.

The boosting is similar to that performed in in vitro or ex vivo in the EV secretion boosting method of the present invention. In the boosting, it is particularly preferable that the cells are caused to coexist with the EV secretion booster in a medium. This allows, as described above, EVs to be secreted from the cells into the medium. Thus, in the subsequent collecting, that is, the collecting of EVs secreted from the cells, the EVs can be easily collected from the medium.

The method for collecting EVs is not particularly limited, and conventionally known methods can be used. Examples of the collecting method include filtration, centrifugal methods such as ultracentrifugation, and precipitation methods. Also, for example, a device in which an antibody against the marker molecule of the EVs mentioned above is immobilized can be used to collect the EVs by causing the EVs to bind to and separate from the antibody.

### <Use>

The present invention is directed to an ABCA1 reducing agent for use in boosting extracellular vesicle secretion from cells. Also, the present invention is directed to an ABCA1 reducing agent for use in producing a secretion booster of extracellular vesicles from cells. As to the ABCA1 reducing agent, reference can be made to the above descriptions regarding the EV secretion booster of the present invention.

### Examples

Next, examples of the present invention will be described. It is to be noted, however, that the present invention is by no means limited by the following examples. Commercially available reagents were used in accordance with their protocols, unless otherwise stated.

### [Materials and Methods]

### (Cells)

A human colorectal carcinoma cell line HCT116 (ATCC CCL-247) was used as colorectal cancer cells, and an adenocarcinomic human alveolar basal epithelial cell line A549 (ATCC CCL-185) was used as lung cancer cells. To confirm versatility, human prostate cancer cells PC3M-Luc-C6 (PerkinElmer CVCL_D577), a human melanoma cell line A375 (ATCC CRL-1619), human fetal kidney cells HEK293 (CRL-1573), a human pancreatic adenocarcinoma cell line PANC-1 (ATCC CRL-1469), human breast cancer cells MM231 (ATCC HTB-26), human normal prostate epithelial cells PNT-2 (KAC Co., Ltd. (formerly known as DS Pharma), EC95012613), adult human female-derived pre-neoplastic breast epithelial cells MCF10A (ATCC CRL-10317) were used. Human bone marrow-derived mesenchymal stem cells BM-MSCs (ATCC PCS-500-012^{™}) and human subcutaneous adipose tissue-derived mesenchymal stem cells AT-MSCs (ATCC PCS-500-011) were used as mesenchymal stem cells.

### (miRNA)

- Example miRNA
   miR-3202 (product No. 4464066, Ambion)
- Negative control miRNA (hereinafter referred to as "NC1")
   miRNA Mimic Negative Control #1 (product No. 4464058, Ambion)

### (Cell Culture)

DMEM (serum-supplemented) and advanced DMEM (serum-free) were used as media for HCT116, A549, PANC-1, A375, and HEK293, and RPMI1640 (serum-supplemented) and advanced RPMI1640 (serum-free) were used as media for PNT2, MM231, and PC3M. MesenPRO RS (serum-free) was used as a medium for BM-MSCs and AT-MSCs. The culture conditions were set to 37°C, 5% carbon dioxide, and 95% relative humidity (RH). MEGM (Kit Catalog No. CC-3150, LONZA, serum-free) was used as a medium for MCF10A.

### (Transfer of miRNA and Collection of Secreted EVs)

In the examples, secreted exosomes were collected and used as secreted EV samples.

For preparation of a secreted EV sample for the ExoScreen method, cells were cultured and supernatant was collected as follows. First, cells were seeded in a 96-well plate using a serum-supplemented medium (the medium according to the type of cells) at 5×10³ cells/medium 0.1 mL/well (Day 0). After incubation for 24 hours (Day 1), 10 fmol of the miRNA was added, and the cells were transfected with the miRNA using a transfection agent (product name Dhermafect1, manufactured by Dhermacon, the same shall apply hereinafter) according to the instructions for use. After further incubation for 24 hours (Day 2), the culture supernatant was removed and replaced with a serum-free new medium (the medium according to the type of cells). Then, after incubation for 48 hours (Day 4), the supernatant was collected from the wells. Then, 10 pL of the collected supernatant was used as a secreted EV sample and the following ExoScreen method was performed.

For preparation of a secreted EV sample for the NTA method, cells were cultured and supernatant was collected and purified as follows. First, cells were seeded in a 6-well dish at 1×10⁵ cells/medium 4 mL/well (Day 0). In the 6-well dish, three wells were for miR-3202, and the remaining three wells were for NC1. After incubation for 24 hours (Day 1), the miRNA was added, and the cells were transfected with the miRNA using the transfection agent according to the instructions for use. After further incubation for 24 hours (Day 2), the culture supernatant was removed and replaced with a serum-free new medium (the medium according to the type of cells). Then, after incubation for 96 hours (Day 6), the supernatant was collected from the wells, and the supernatants from the three wells with the same miRNA were mixed to obtain approximately 12 mL of mixed supernatant. After collecting the supernatant, the number of cells in each well was counted. Next, the collected supernatant mixture was centrifuged at 2000 ×g for 10 minutes to remove cell debris, and then filtered through a 0.22 pm filter (Millipore) to collect filtrate. Then, EVs (exosomes) were purified through ultracentrifugation using 11 mL of the filtrate. Specifically, the filtrate was centrifuged at 110,000 ×g for 70 minutes to obtain pellets in which secreted EVs were concentrated. The pellets were washed with 11 mL of PBS, ultracentrifuged at 110,000 ×g for 70 minutes, and collected again. The resultant was used as the secreted EV sample and subjected to the following NTA method.

### (ExoScreen Method)

EV detection was performed using an exosome marker CD9 or C63 according to the ExoScreen method. EV detection was performed using an AlphaLISA reagent (Perkin Elmer) composed of AlphaScreen streptavidin-coated donor beads (6760002), AlphaLISA unconjugated acceptor beads (6062011), and an AlphaLISA universal buffer (AL001F), a 96-well half-area white plate (6005560, Perkin Elmer), and a detection device EnSpire Alpha 2300 Mutilabel plate reader (Perkin Elmer). Specifically, 10 µL of secreted EV sample for the ExoScreen method, 10 pL of a 5 nmol/L biotinylated antibody solution prepared using the above-mentioned buffer, and 10 pL of 50 pg/mL AlphaLISA acceptor bead-conjugated antibody were added to each well of the plate. For detection of CD9/CD9 double-positive EVs, a biotinylated anti-human CD9 antibody and an anti-human CD9 antibody conjugated with AlphaLISA acceptor beads were used. For detection of CD63/CD63 double-positive EVs, a biotinylated anti-human CD63 antibody and an anti-human CD63 antibody conjugated with AlphaLISA acceptor beads were used. The plate was incubated at 37°C for 1 hour, and then, 25 pL of 80 pg/mL AlphaScreen streptavidin-coated donor beads were added thereto. The plate was incubated at 37°C for another 30 minutes in the dark. Next, luminescence in the wells of the plate was measured using the detection device with the excitation wavelength set to 680 nm and the emission detection wavelength set to 615 nm. The antibodies used were both commercially available products, namely, a mouse monoclonal anti-human CD9 (Clone 12A12) and a CD63 antibody (Clone 8A12) (both available from Cosmo Bio).

### (Analysis of EVs by Nanoparticle Tracking Analysis (NTA))

The secreted EV sample for the NTA method was suspended in PBS. Further, a diluted system was prepared using PBS and subjected to NanoSight particle tracking analysis (LM10, software ver. 2.03). In the particle tracking, at least five 60-second videos were taken for each sample with the camera level set to 14. Analysis settings were optimized and kept constant between the samples. An EV concentration was calculated as particles/cells in the culture solution, whereby a net EV secretion rate was obtained. The results of EV measurement by the ExoScreen method correlated with the results of EV measurement by the NTA method. This confirmed that the ExoScreen method is capable of measuring the amount of secreted EVs.

Unless otherwise stated, data presented in the examples were each expressed as the mean value ± the standard error. The statistical significance was determined using a Student's t-test. In dot plots, each bar indicates the median and the interquartile range, and the statistical significance was determined by a Student's t-test. P < 0.05 was considered as statistically significant.

### * P < 0.05, ** P < 0.01.

### [Example A1]

It was confirmed whether or not miR-3202 boosts EV secretion.

### (1) Boosting of EV Secretion from HCT and A549

HCT116 andA549 were transfected with miR-3202 using the method described above, secreted EV samples were prepared from the collected culture supernatant, and the amount of secreted EVs was measured according to the ExoScreen and NTA methods described above. The amount of secreted EVs in the negative control (NC) was measured in the same way, except that negative control miRNA (the NC1) was used. The relative values of secreted EVs in the example (n=3) were then determined assuming that the amount of secreted EVs in the NC was 1.

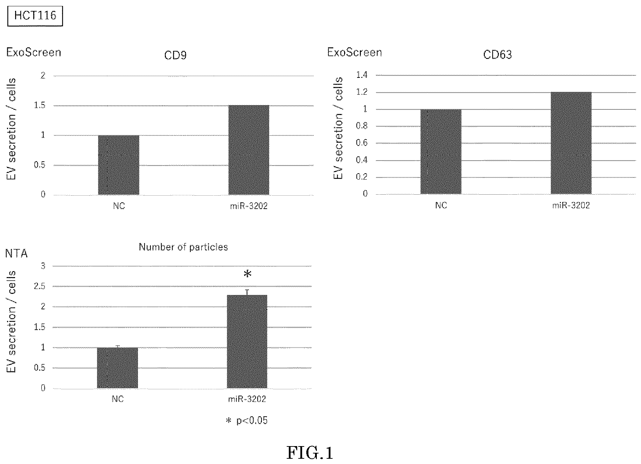

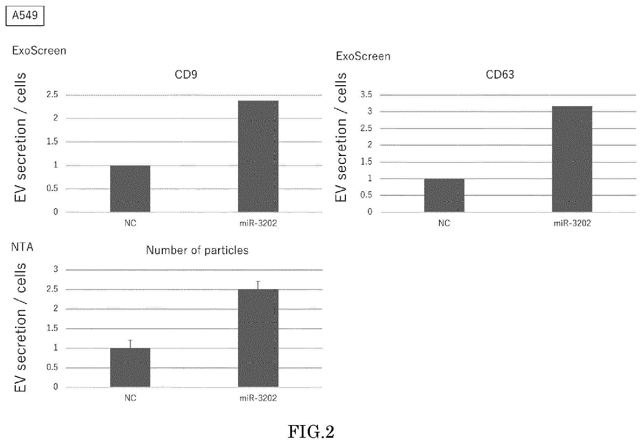

FIGS. 1 and 2 show the results. FIG. 1 shows the results of the amount of secreted EVs of the transfected HCT116, and FIG. 2 shows the results of the amount of secreted EVs of the transfected A549. In FIGS. 1 and 2, the upper left graphs show the results of CD9/CD9 double-positive EVs according to ExoScreen, the upper right graphs show the results of CD63/CD63 double-positive EVs according to ExoScreen, and the lower graphs show the results according to the NTA method. As shown in FIGS. 1 and 2, it was confirmed that, in both HCT116 and A549, transfection with miR-3202 increased the amount of secreted EVs compared with that of the NC, that is, significantly boosted the EV secretion. In addition, it was confirmed from immunofluorescence staining that the amount of EV marker CD63 increased in the cytoplasm of cells cultured in the presence of miR-3202.

### (2) Boosting of EV Secretion from Other Cells

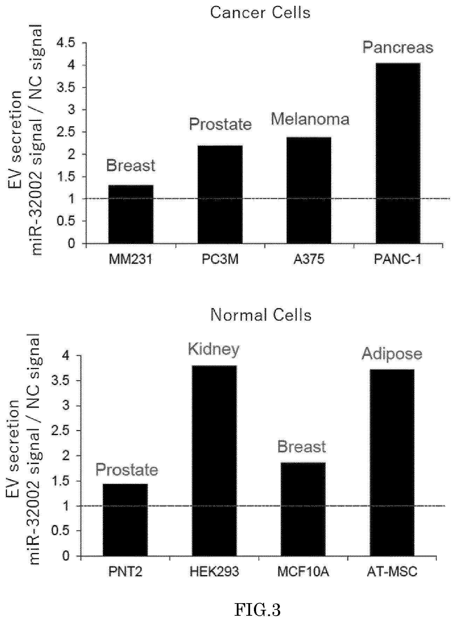

As shown in (1) above, it was found that EV secretion from cells was boosted by miR-3202 in both HCT116 and A549. Therefore, it was confirmed whether or not EV secretion can be boosted by miR-3202 with wide versatility regardless of the type of cells. The amount of secreted EVs was measured in the same way as that in (1) above, except that the cells shown in FIG. 3 were used. FIG. 3 shows the results. FIG. 3 shows the results of CD63-positive EVs according to ExoScreen, expressed in terms of a relative value of signal intensity with respect to a measured value of the NC.

As shown in FIG. 3, the relative value exceeded 1 in all cells. It was confirmed from these results that miR-3202 can boost EV secretion from cells regardless of the type of cells.

### (3) EVs Obtained by Boosting Secretion

An EV sample (miR-3202) collected from cells cultured in the presence of miR-3202 was compared with an EV sample (C) collected from cells cultured in the absence of miR-3202.

Confirmation on EV markers (CD9, CD63, CD81, and TSG101) and endosome markers (RAB5, EEA1, LAMP1, and RAB7) according to Western blotting for the EV sample (miR-3202) and the EV sample (C) showed that both markers were detected in the two samples, and there was no significant difference between them.

Furthermore, the function of the EV sample collected from cells cultured in the presence of miR-3202 was compared with that of the EV sample collected from cells cultured in the absence of miR-3202. Specifically, the EV sample (miR-3202) was collected from BM-MSCs cultured in the presence of miR-3202, and the EV sample (C) was collected from BM-MSCs cultured in the absence of miR-3202. Then, HEL293 was cultured in the absence of the EV sample, in the presence of the EV sample (miR-3202), and in the presence of the EV sample (C), and their cell proliferative abilities were compared by counting the number of cells. The results showed that compared with that of the cells cultured in the absence of the EV sample, there was a significant increase in the cell proliferative ability in the cells with the EV sample (miR-3202) and the EV sample (C), but there was no significant difference between that of cells cultured in the presence of the EV sample (miR-3202) and in the presence of the EV sample (C). This indicates that EVs obtained when EV secretion is boosted by miR-3202 for the target cells are similar to those obtained when the target is not in contact with miR-3202.

### (3) Analysis of Secreted EVs

For the secreted EV samples obtained in (1) above, the size of EVs contained in the samples was confirmed. The secreted EV samples were submitted to a nanoparticle analysis system (product name NanoSight, Malvern) to measure the particle size distribution of the particles (EVs) contained in the secreted EV samples.

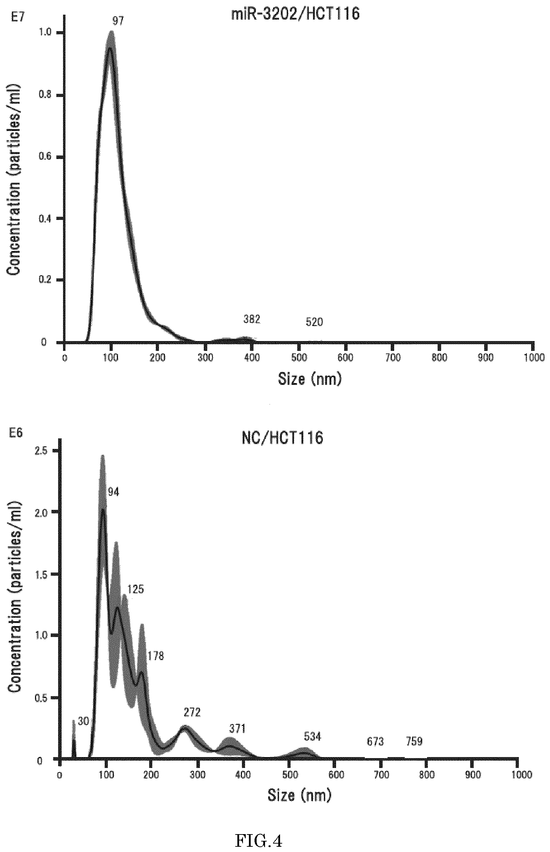

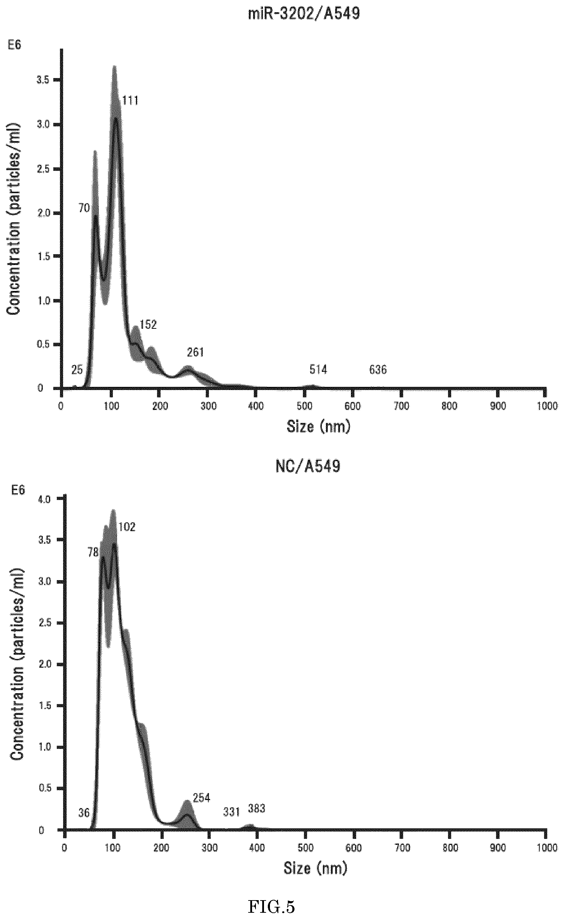

FIGS. 4 and 5 show the results of the particle size distribution. FIG. 4 shows the results of the secreted EV samples secreted from HCT116, where the upper graph shows the results of the EV sample secreted from cells transfected with miR-3202, and the lower graph shows the results of the EV sample secreted from the NC (negative control). FIG. 5 shows the results of the secreted EV samples secreted from A549, where the upper graph shows the results of the EV sample secreted from cells transfected with miR-3202, and the lower graph shows the results of the EV sample secreted from the NC (negative control). In FIGS. 4 and 5, the vertical axes indicate particle concentration (particles/mL) and the horizontal axes indicate particle size (nm), where the thin lines indicate the average concentration of three measurements with respect to the particle size, and the thick lines indicate each concentration (concentration range) obtained in three measurements with respect to the particle size. In HCT116 in FIG. 4, the particle size peak (97 nm) of the secreted EV sample of the cells transfected with miR-3202 was comparable to the particle size peak (94 nm) of the secreted EV sample of the NC. Similarly, in A549 in FIG. 5 as well, the particle size peak (111 nm) of the secreted EV sample of the cells transfected with miR-3202 was comparable to the particle size peak (102 nm) of the secreted EV sample of the NC. It was confirmed from these results that the EVs released from the cells transfected with miR-3202 are similar to those of the control.

### (4) Confirmation that Increase in Amount of EVs is due to Boosting of Secretion

If cell death by apoptosis occurs, apoptotic bodies may be released. It was confirmed whether or not the EVs secreted from cells are not apoptotic bodies.

Caspase activity is a known marker for apoptosis. Therefore, the caspase activity of cell samples obtained by culturing various types of cells transfected with the miRNAs in (1) and (2) above was measured. The cell viability of the cell samples was also measured by MTS assay. The caspase activity of the cell samples was then normalized by the respective cell viability rates, and the normalized caspase activity of the cells transfected with the miR-3202 was divided by the normalized caspase activity of the cells transfected with the NC1 to obtain a relative value of caspase activity (miR-3202/NC1).

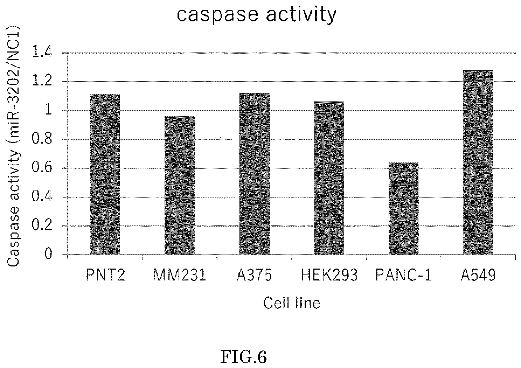

FIG. 6 shows the results. FIG. 6 shows the caspase activity of the cells, where the vertical axis indicates a relative value of caspase activity (miR-3202INC1). If the relative value of caspase activity is about 1 or less than 1, it can be concluded that caspase activity is not increased by the transfection with miRNA. As shown in FIG. 6, the relative value of the caspase activity was about 1 or less than 1 in all cells, and the activity of caspase was not significantly increased in the cells transfected with miR-3202 compared with that of cells transfected with the control NC1. Thus, it was seen that the EV secretion booster of the present invention can, for example, maintain cell survival and boost EV secretion, rather than promoting cell death by apoptosis. Thus, the EVs in the present invention can also be said to be EVs derived from viable cells.

### [Example A2]

It was confirmed whether or not miR-3202 boosts EV secretion from mesenchymal stem cells.

In Example A1 above, it was confirmed that miRNAs in the present invention can boost EV secretion from cells with wide versatility. Therefore, it was further confirmed whether or not EV secretion from mesenchymal stem cells derived from bone marrow is boosted.

### (1) Boosting of EV Secretion from Bone Marrow-Derived MSCs

BM-MSCs were transfected with miR-3202, secreted EV samples were prepared from the collected culture supernatant, and the amount of secreted EVs was measured according to the NTA method, in the same way as that of Example A1, except that BM-MSCs were used as cells and MesenPRO RS (serum-free) was used as a medium. The amount of secreted EVs in the negative control (NC) was measured in the same way, except that the negative control miRNA (NC1) was used. The relative values of secreted EVs in the example (n=3) were then determined assuming that the amount of secreted EVs in the NC was 1. The secreted EV samples were submitted to the nanoparticle analysis system (product name NanoSight, Malvern) to measure the particles (EVs) contained in the secreted EV samples.

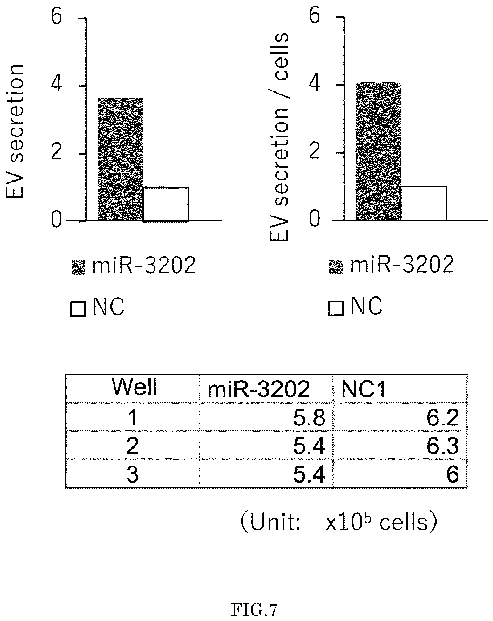

FIG. 7 shows the results. In FIG. 7, the left graph shows the results of the amount of secreted EVs of the transfected MSC, as measured using the nanoparticle analysis system, the right graph shows the results of the amount of secreted EVs of the transfected MSC cells, as measured according to the NTA method, and the lower table shows the results of counting the number of cells in each well (n=3) for the cells transfected with miRNA.

The amount of secreted EVs measured according to the NTA method is normalized by the number of viable cells. Therefore, assuming that apoptosis has been caused by transfection with miR-3202, the amount of secreted EVs measured using the nanoparticle analysis system, which is not normalized by the number of viable cells, and the amount of secreted EVs measured according to the NTA method would yield different results. However, as shown in the graphs in FIG. 7, the amount of secreted EVs measured using the nanoparticle analysis system and the amount of secreted EVs measured according to the NTA method yielded similar results. In addition, as shown in the table in FIG. 7, as a result of a comparison between the number of cells of the BM-MSCs transfected with NC1 as miRNA and that of the BM-MSCs transfected with miR-3202, no significant decrease in the number of cells due to transfection with miR-3202 was confirmed. These results indicate that transfection with miR-3202 did not cause apoptosis of the BM-MSCs, and that a significant increase in the amount of secreted EVs in the BM-MSCs transfected with miR-3202 compared with that of the NC seems to be due to boosting of EV secretion by miR-3202.

### (2) Analysis of Secreted EVs

For the secreted EV samples obtained in (1) above, the size of EVs contained in the samples was confirmed as in (3) of Example A1 above.

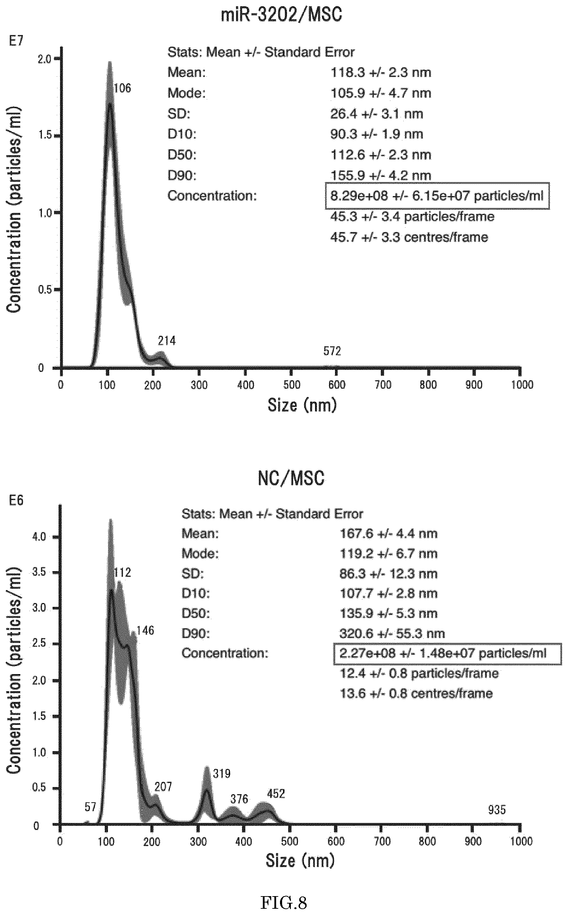

FIG. 8 shows the results of the particle size distribution. FIG. 8 shows the results of the secreted EV samples secreted from the BM-MSCs, where the upper graph shows the results of the EV sample secreted from cells transfected with miR-3202, and the lower graph shows the results of the EV sample secreted from the NC (negative control). In FIG. 8, the vertical axes indicate particle concentration (particles/mL) and the horizontal axes indicate particle size (nm), where the thin lines indicate the average concentration of three measurements with respect to the particle size, and the thick lines indicate each concentration (concentration range) obtained in three measurements with respect to the particle size. As shown in FIG. 8, the particle size peak (106 nm) of the secreted EV sample of the cells transfected with miR-3202 was comparable to the particle size peak (112 nm) of the secreted EV sample of the NC. It was confirmed from these results that the EVs released from the BM-MSCs transfected with miR-3202 are similar to those of the control.

### [Example A3]

It was confirmed whether or not miR-3202 reduces expression of the ABCA1 gene.

A549, HCT116, and Panc6 were transfected with miR-3202 in the same way as that of Example A2 above and cultured, and then the expression level of ABCA1 mRNAin the obtained cultured cells was measured. The ABCA1 mRNA was measured through RT-PCR. As a negative control, cells were cultured and expression levels were measured in the same way, except that the miR-3202 was not added. The relative values of the expression level of cells transfected with miR-3202 were then determined assuming that the expression level in the negative control was a relative value of 1.

As a result, the relative value of the expression level in A549 was 0.61, the relative value of the expression level in HCT116 was 0.63, and the relative value of the expression level in Panel was 0.63, that is, it was confirmed that the relative value was less than 1 in all cells, and the expression of the ABCA1 gene was reduced compared with that of the NC. It was confirmed from these results that the target gene of miR-3202 is the ABCA1 gene. Furthermore, since it was confirmed in the foregoing examples that EV secretion can be boosted by miR-3202, it can be concluded that EV secretion can be boosted not only by miR-3202 but also by reducing the expression of the ABCA1 gene, which is the target gene of miR-3202, or by reducing the function of the ABCA1 protein that is encoded by the target gene.

### [Example B]

It was confirmed in Example A above that miR-3202, which reduces expression of ABCA1, boosts expression of EVs. Thus, it was confirmed whether or not an ABCA1 reducing agent other than miR-3202 can boost EV secretion in a similar manner.

### [Example B1]

It was confirmed whether or not knockdown of the ABCA1 gene boosts EV secretion.

### (1) Reducing Expression of ABCA1 Gene by siRNA

The following two siRNAs were used as siRNAs against the ABCA1 gene.
siABCA1_1: product name siGENOME siRNA Human ABCA1 (D-004128-01-0002), Dharmacon
siABCA1_2: product name siGENOME siRNA Human ABCA1 (D-004128-03-0002), Dharmacon

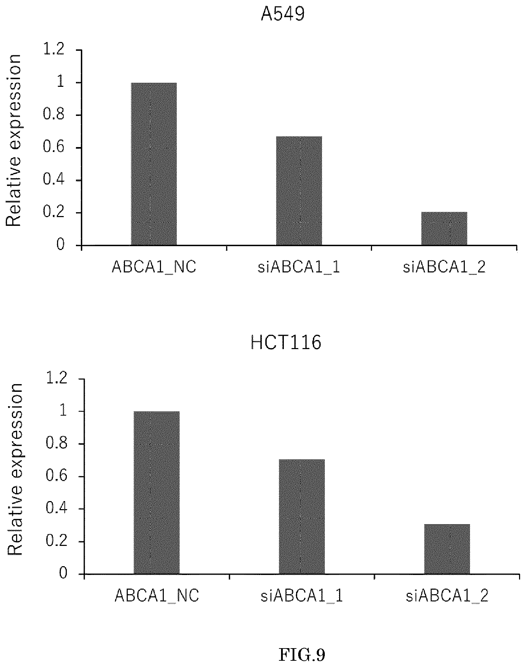

A549 and HCT116 were transfected with the siRNA and cultured, and then the expression level of ABCA1 mRNA in the obtained cultured cells was measured. The cells were cultured as described in [Materials and Methods] above, and the transfection with the siRNA was performed in the same way as the transfer of miRNAin [Materials and Methods] above. The ABCA1 mRNA was measured through RT-PCR. As a negative control, cells were cultured and expression levels were measured in the same way, except that the siRNA was not added. The relative values of the expression level of cells transfected with siRNA were then determined assuming that the expression level in the negative control was a relative value of 1. FIG. 9 shows the results.

FIG. 9 shows graphs of the relative values of the expression level of ABCA1 mRNAin cells transfected with siRNA against the ABCA1 gene, where the upper graph shows the results of A549 cells, and the lower graph shows the results of HCT116 cells. As shown in FIG. 9, the expression level of ABCA1 mRNAin A549 and HCT116 significantly decreased compared with that of the negative control regardless of which of siABCA1_1 and siABCA1_2 was used. It was confirmed from these results that the ABCA1 gene was knocked out by siRNA for ABCA1.

### (2) Boosting of EV Secretion by Reducing Expression of ABCA1 Gene

A549 and HCT116 were transfected with the siRNA and cultured in the same way as that in (1) above. After confirming the number of cells after culture, secreted EV samples were prepared from the collected culture supernatant and the amount of secreted EVs was measured according to the NTA method as described in [Materials and Methods] above. The negative control (NC) was similar to that in (1) above, and the amount of secreted EVs therein was measured. The relative value of secreted EVs in the cells transfected with siRNA was then determined assuming that the amount of secreted EVs in the NC was a relative value of 1, and the relative value of the number of cells in the cells transfected with siRNA was calculated as cell viability assuming that the number of cells in the NC was a relative value of 1.

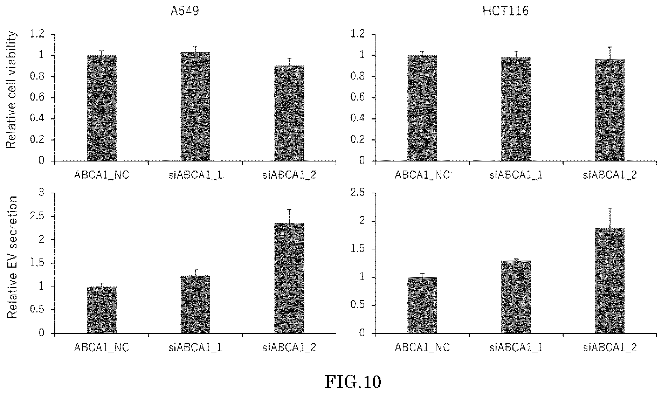

FIG. 10 shows the results. FIG. 10 shows results of cells transfected with siRNA against the ABCA1 gene, where the upper graphs are graphs of the cell viability, the lower graphs are graphs of the relative values of the amount of secreted EVs, the left graphs show the results of A549 cells, and the right graphs show the results of HCT116 cells. As shown in the upper graphs in FIG. 10, in both A549 cells and HCT116 cells, no decrease in the viability due to transfection with siRNA was observed compared with that of the NC. On the other hand, as shown in the lower graphs in FIG. 10, in both A549 cells and HCT116 cells, an increase in the EV secretion due to transfection with siRNA was confirmed compared with that of the NC. In particular, the EV secretion significantly increased due to transfection with siABCA1_2. It was confirmed from these results that knockout of the ABCA1 gene can boost EV secretion without affecting cell survival.

### [Example B2]

It was confirmed whether or not an inhibitor of the ABCA1 protein boosts EV secretion.

Cyclosporin, probucol, and valspodar were used as inhibitors. A549 and HCT116 were cultured as described in (Transfer of miRNA and Collection of Secreted EVs) in [Materials and Methods] above. After incubation for 24 hours (Day 1), the number of cells was counted and the amount of secreted EVs was measured in the same way, except that the inhibitors were added instead of the miRNA. Each of the inhibitors was added at 10 nmol/L or 100 nmol/L per well. As a negative control, the cells were cultured, the number of cells was counted, and the amount of secreted EVs was measured in the same way, except that the inhibitors were not added. The relative value of secreted EVs in the cells to which the inhibitors were added was then determined assuming that the amount of secreted EVs in the NC was a relative value of 1, and the relative value of the number of cells in the cells to which the inhibitors were added was calculated as cell viability assuming that the number of cells in the NC was a relative value of 1.

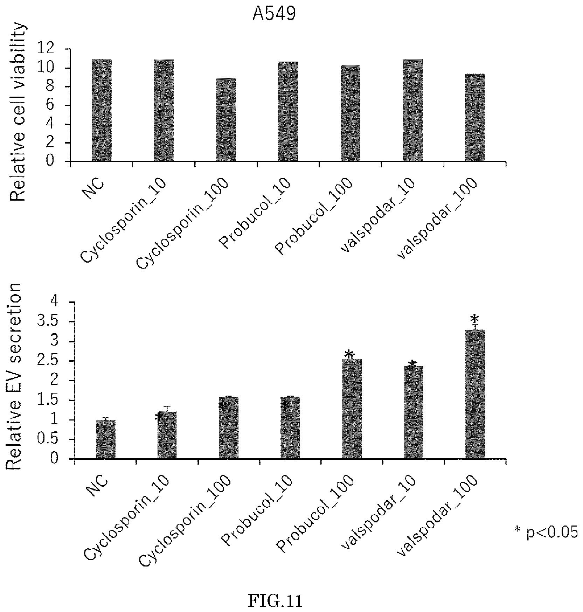

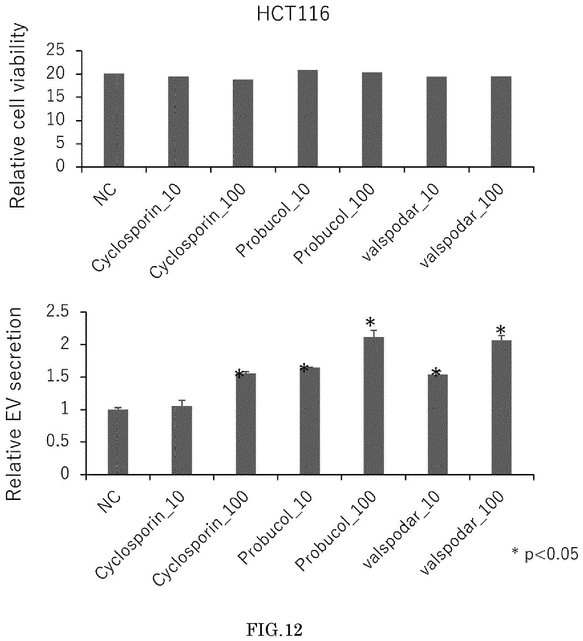

FIGS. 11 and 12 show the results. FIG. 11 shows the results of A549 cells to which the inhibitors were added, where the upper graph is a graph of the cell viability, and the lower graph is a graph of the relative values of the amount of secreted EVs. FIG. 12 shows the results of HCT116 cells to which the inhibitors were added, where the upper graph is a graph of the cell viability, and the lower graph is a graph of the relative values of the amount of secreted EVs. As shown in the upper graphs in FIGS. 11 and 12, in both A549 cells and HCT116 cells, no significant decrease in the viability due to addition of the inhibitors was observed compared with that of the NC. On the other hand, as shown in the lower graphs in FIGS. 11 and 12, in both A549 cells and HCT116 cells, an increase in the EV secretion due to addition of the inhibitors was confirmed compared with that of the NC. In addition, EV secretion was boosted in a concentration-dependent manner regardless of which inhibitor was used. Among the inhibitors, probucol and valspodar particularly effectively boosted EV secretion.

While the present invention has been described above with reference to illustrative embodiments and examples, the present invention is by no means limited thereto. Various changes and modifications that may become apparent to those skilled in the art may be made in the configuration and specifics of the present invention without departing from the scope of the present invention.

This application claims priority from Japanese Patent Application No. 2021-166634 filed on October 11, 2021. The entire disclosures of this Japanese patent application are incorporated herein by reference.

### Industrial Applicability

According to the present invention, extracellular vesicle secretion from cells can be boosted. Thus, for example, the preparation of extracellular vesicles as a material and the secretion of endogenous extracellular vesicles in a living organism by causing extracellular vesicle secretion from cells can be efficiently performed. Therefore, it can be said that the present invention, for example, is very useful in the medical field.

## Claims

1. An extracellular vesicle secretion booster, comprising:
an ABCA1 reducing agent.

2. The extracellular vesicle secretion booster according to claim 1, wherein the ABCA1 reducing agent is an expression reducing substance or a function reducing substance against an ABCA1 protein.

3. The extracellular vesicle secretion booster according to claim 2, wherein the expression reducing substance is at least one substance selected from the group consisting of a substance that reduces transcription from a gene encoding an ABCA1 protein, a substance that degrades a transcription product resulting from transcription, and a substance that reduces translation of a transcription product into a protein.

4. The extracellular vesicle secretion booster according to claim 3, wherein the expression reducing substance is at least one nucleic acid substance selected from the group consisting of an miRNA, an siRNA, an antisense, and a ribozyme, and a precursor thereof.

5. The extracellular vesicle secretion booster according to claim 4, wherein the expression reducing substance is an expression vector that expresses the nucleic acid substance.

6. The extracellular vesicle secretion booster according to claim 2, wherein the function reducing substance is a function inhibitory substance or a function neutralizing substance against the ABCA1 protein.

7. The extracellular vesicle secretion booster according to claim 6, wherein the function neutralizing substance is an antibody or an antigen-binding fragment against the ABCA1 protein.

8. The extracellular vesicle secretion booster according to claim 7, wherein the function reducing substance is an expression vector that expresses the function neutralizing substance.

9. The extracellular vesicle secretion booster according to claim 4, wherein the expression reducing substance is miR-3202 or a precursor thereof.

10. The extracellular vesicle secretion booster according to claim 9, wherein the precursor is at least one nucleic acid substance selected from the group consisting of a nucleic acid encoding the miRNA, a primary miRNA transcription product, the precursor miRNA, and an expression vector of the miRNA.

11. The extracellular vesicle secretion booster according to any one of claims 1 to 10,
wherein the extracellular vesicle secretion booster is for boosting extracellular vesicle secretion from a cell, and
the cell is a mesenchymal stem cell.

12. An extracellular vesicle secretion boosting method comprising:
boosting extracellular vesicle secretion by causing a target having a cell to coexist with the extracellular vesicle secretion booster according to any one of claims 1 to 11.

13. The secretion boosting method according to claim 12, wherein, in the boosting, the extracellular vesicle secretion booster is added to the target in vivo, ex vivo, or in vitro.

14. The secretion boosting method according to claim 12 or 13, wherein, in the boosting, the cell is transfected with the extracellular vesicle secretion booster.

15. The secretion boosting method according to any one of claims 12 to 14, wherein the cell is a mesenchymal stem cell.

16. The secretion boosting method according to claim 15, wherein the mesenchymal stem cell is a mesenchymal stem cell derived from a bone marrow or an adipose tissue.

17. The secretion boosting method according to any one of claims 12 to 16, wherein the cell is a cell collected from a living organism or a cell line.

18. The secretion boosting method according to claim 17, wherein, in the boosting, the cell is caused to coexist with the extracellular vesicle secretion booster in a medium.

19. The secretion boosting method according to any one of claims 12 to 18, wherein the target is a tissue, an organ, or a living organism.

20. A method for producing an extracellular vesicle, comprising:
boosting extracellular vesicle secretion by causing a cell to coexist with the extracellular vesicle secretion booster according to any one of claims 1 to 11; and
collecting an extracellular vesicle secreted from the cell.

21. The production method according to claim 20, wherein the cell is transfected with the extracellular vesicle secretion booster.

22. The production method according to claim 20 or 21, wherein the cell is a mesenchymal stem cell.

23. The production method according to claim 22, wherein the cell is a mesenchymal stem cell derived from a bone marrow or an adipose tissue.

24. The production method according to any one of claims 20 to 23, wherein the cell is a cell collected from a living organism or a cell line.

25. The production method according to any one of claims 20 to 24, wherein, in the boosting, the cell is caused to coexist with the extracellular vesicle secretion booster in a medium.

26. The production method according to any one of claims 20 to 25, wherein the method is for producing the extracellular vesicle as a carrier in a drug delivery system.

27. The production method according to any one of claims 20 to 25, wherein the method is for producing the extracellular vesicle as a therapeutic agent.

28. An ABCA1 reducing agent for use in boosting extracellular vesicle secretion from a cell.
